(19) **Europäisches Patentamt**
**European Patent Office**
**Office européen des brevets**

(11) **EP 3 437 627 B2**

(12) # NEUE EUROPÄISCHE PATENTSCHRIFT
Nach dem Einspruchsverfahren

(45) Veröffentlichungstag und Bekanntmachung des Hinweises auf die Entscheidung über den Einspruch:
**05.03.2025 Patentblatt 2025/10**

(45) Hinweis auf die Patenterteilung:
**01.04.2020 Patentblatt 2020/14**

(21) Anmeldenummer: **18181901.2**

(22) Anmeldetag: **05.07.2018**

(51) Internationale Patentklassifikation (IPC):
*A61K 8/35* (2006.01)          *A61K 8/37* (2006.01)
*A61K 8/49* (2006.01)          *A61Q 17/04* (2006.01)
*A61K 8/34* (2006.01)

(52) Gemeinsame Patentklassifikation (CPC):
**A61K 8/37; A61K 8/34; A61K 8/35; A61K 8/4946; A61K 8/4966; A61Q 17/04**

(54) **2-PROPYLHEPTYLOCTANOAT IN KOSMETISCHEN ZUBEREITUNGEN**

2-PROPYLHEPTYLOCTANOAT IN COSMETIC PREPARATIONS

2-PROPYLHEPTYLOCTANOATE DANS LES PRÉPARATIONS COSMÉTIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **01.08.2017 DE 102017213232**

(43) Veröffentlichungstag der Anmeldung:
**06.02.2019 Patentblatt 2019/06**

(73) Patentinhaber: **BASF SE**
**67056 Ludwigshafen am Rhein (DE)**

(72) Erfinder:
• **Eitrich, Anja**
**25337 Kölln-Reisiek (DE)**
• **Deinhard, Sybil-Marie**
**22307 Hamburg (DE)**
• **Schade, Juliane**
**22529 Hamburg (DE)**

(74) Vertreter: **BASF IP Association**
**BASF SE**
**GBI - Z078**
**67056 Ludwigshafen (DE)**

(56) Entgegenhaltungen:
EP-A1- 3 093 006          EP-A1- 3 173 129
EP-A1- 3 173 130          EP-A2- 1 180 359
EP-A2- 2 837 407          EP-B1- 3 093 005
EP-B1- 3 093 008          DE-A1- 102007 017 436

• **DATABASE GNPD [online] MINTEL; 17 May 2017 (2017-05-17), ANONYMOUS: "Shimmering Sun Lotion SPF 20", XP055504401, retrieved from www.gnpd.com Database accession no. 4821013**
• **"Cosmetic and dermatologic formulations containing the polymeric rheology modifier Polyurethane-39", IP.COM JOURNAL, IP.COM INC., WEST HENRIETTA, NY, US, 8 June 2009 (2009-06-08), XP013132198, ISSN: 1533-0001**

Bemerkungen:
Die Akte enthält technische Angaben, die nach dem Eingang der Anmeldung eingereicht wurden und die nicht in dieser Patentschrift enthalten sind.

EP 3 437 627 B2

**Beschreibung**

Die vorliegende Erfindung betrifft die Verwendung von 2-Propylheptyloctanoat (INCI Propylheptyl Caprylate) in kosmetischen Zubereitungen zur Reduzierung der Sand anhaftenden Eigenschaften der Zubereitung und Zubereitungen die neben 2-Propylheptyloctanoat (INCI Propylheptyl Caprylate) auch Phenoxyethanol, Ethanol und mindestens einen im UV-A Bereich absorbierenden organischen UV-Filter enthalten.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen:
Kosmetische Sonnenschutzmittel weisen unter anderem aufgrund ihres UV-Filtergehaltes meist eine gewisse Klebrigkeit auf, die insbesondere bei der Verwendung am Strand dazu führt, dass es auf eingecremten Hautpartien zur Anhaftung von Sand kommt. Dieses Problem wird umso größer, je mehr UV-Filter eine Zubereitung enthält. Zwar hat es in der Vergangenheit nicht an Versuchen gefehlt, sandabweisende Sonnenschutzmittel zu entwickeln, doch ist dieses Problem besonders bei Zubereitungen mit hohem Lichtschutzfaktor bis heute nicht endgültig zufriedenstellend gelöst.

Es war daher die Aufgabe der vorliegenden Erfindung, ein Sonnenschutzmittel mit reduzierter Sandanhaftung ("sandabweisend") zu entwickeln. Insbesondere sollte ein Sonnenschutzmittel mit hohem Lichtschutzfaktor (SPF 50 und höher) entwickelt werden, welches besonders wenig sandanhaftend ist.

Überraschend gelöst wird die Aufgabe durch eine Verwendung nach Anspruch 1 bis 3.

Darüber hinaus wird die Aufgabe überraschend gelöst durch eine kosmetische Zubereitung nach Anspruch 4.

Zwar kennt der Stand der Technik Sonnenschutzmittel mit 2-Propylheptyloctanoat (INCI Propylheptyl Caprylate), beispielsweise die DE 102007017436.7 oder die 102007017440.5, doch konnten diese Schriften nicht den Weg zur vorliegenden Erfindung weisen, da die Bedeutung von 2-Propylheptyloctanoat auf die Klebrigkeit und, speziell auf die Sandanhaftung unentdeckt blieb.

Außerdem kennt der Stand der Technik den Datenbankeintrag in der Mintel-Datenbank GNPD, "Shimmering Sun Lotion SPF", Record ID 4821013, sowie die Dokumente ip.com Prior Art Database, IP.com Number IPCOM000183957D, sowie die EP3093006 A1, die ebenfalls nicht den Weg zur vorliegenden Erfindung weisen konnten.

Die Formulierungen "erfindungsgemäß", "erfindungsgemäß vorteilhaft", "vorteilhaft im Sinne der Vorliegenden Erfindung" etc. beziehen sich im Rahmen der vorliegenden Offenbarung immer sowohl auf die erfindungsgemäßen Verwendungen als auch auf die erfindungsgemäße Zubereitung.

Die erfindungsgemäße Zubereitung enthält 2-Propylheptyloctanoat (INCI Propylheptyl Caprylate) in einer Konzentration von 1 bis 5 Gewichts-%, bezogen auf das Gesamtgewicht der Zubereitung, enthält. Dabei ist ein Gehalt von 1 bis 3,5 Gewichts-%, bezogen auf das Gesamtgewicht der Zubereitung, erfindungsgemäß bevorzugt.

Erfindungsgemäß bevorzugt werden der oder die im UV-A Bereich absorbierenden organischen UV-Filter gewählt werden aus der Gruppe der Verbindungen 4-(tert.-Butyl)-4'-methoxydibenzoylmethan, Hexyl 2-[4-(diethylamino)-2-hydroxybenzoyl]benzoate (INCI: Diethylamino hydroxybenzoyl hexyl benzoate) und/oder 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine).

Erfindungsgemäß besonders bevorzugt ist dabei eine Kombination aus 4-(tert.-Butyl)-4'-methoxydibenzoylmethan und 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine).

Die erfindungsgemäß vorteilhaften Einsatzkonzentrationen betragen für 4-(tert.-Butyl)-4'-methoxydibenzoylmethan von 1 bis 5 Gewichts-%, für Hexyl 2-[4-(diethylamino)-2-hydroxybenzoyl]benzoate (INCI: Diethylamino hydroxybenzoyl hexyl benzoate) von 1 bis 5 Gewichts-% und für 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine) von 1 bis 5 Gewichts-%, jeweils bezogen auf das Gesamtgewicht der Zubereitung.

Vorteilhaft im Sinne der vorliegenden Erfindung ist es, wenn die erfindungsgemäße Zubereitung Phenoxyethanol in einer Konzentration von 0,2 bis 1,0 Gewichts-%, bezogen auf das Gesamtgewicht der Zubereitung, enthält.

Erfindungsgemäß vorteilhafte Ausführungsformen der vorliegenden Erfindung sind ferner dadurch gekennzeichnet, dass die Zubereitung Ethanol in einer Konzentration von 0,5 bis 20 Gewichts-%, bezogen auf das Gesamtgewicht der Zubereitung, enthält.

Darüber hinaus kann die erfindungsgemäße Zubereitung vorteilhaft einen oder mehrere weitere UV-Filter gewählt aus der Gruppe der Verbindungen Salicylsäure-2-ethylhexylester (INCI Octyl Salicylate), 3,3,5-Trimethylcyclohexyl 2-hydroxybenzoat (INCI: Homosalate), 2,4,6-Tris-[anilino-(p-carbo-2'-ethyl-1'-hexyloxy)]-1,3,5-triazin (INCI: Ethylhexyl Triazone) und/oder 2-Phenylbenzimidazol-5-sulfonsäuresalze, enthalten.

Darüber hinaus ist es erfindungsgemäß von Vorteil, wenn Verwendung die erfindungsgemäße Zubereitung keine Polyethylenglycolether oder Polyethylenglycolester enthält.

Die erfindungsgemäße Zubereitung kann erfindungsgemäß in unterschiedlichen Formen vorliegen. Die erfindungsgemäß bevorzugten Formen sind dadurch gekennzeichnet, dass die Zubereitung in Form einer O/W-Emulsion vorliegt.

Liegt die erfindungsgemäße Zubereitung in Form einer O/W-Emulsion vor, so ist sie erfindungsgemäß vorteilhaft dadurch gekennzeichnet, dass die Zubereitung einen oder mehrere O/W-Emulgatoren gewählt aus der Gruppe der Verbindungen Glycerylstearatcitrat, Glycerylstearat (selbstemulgierend), Stearinsäure, Stearatsalze, Polyglyceryl-3-methylglycosedistearat, Natriumcetearylsulfat, Kaliumcetylphosphat, Polyglyceryl-10 Stearate , Natriumstearylglutamat, enthält.

Es ist erfindungsgemäß bevorzugt, wenn die Zubereitung Natriumstearylglutamat als Emulgator enthält.

Darüber hinaus sind die erfindungsgemäß vorteilhaften Ausführungsformen der vorliegenden Erfindung dadurch gekennzeichnet, dass die Zubereitung ein oder mehrere Alkandiole aus der Gruppe der Verbindungen 1,2-Pentandiol, 1,2-Hexandiol, 1,2-Octandiol, 1,2-Decandiol, 2-Methyl-1,3-propandiol enthält.

Nicht zuletzt erhält man erfindungsgemäß besonders vorteilhafte Ausführungsformen, wenn die Zubereitung eine oder mehrere Öle gewählt aus der Gruppe der Verbindungen Di-n-butyladipat, Dicaprylylcarbonat, C12-C15 Alkylbenzoat, und/oder Butylene Glycol Dicaprylate/Dicaprate, enthält.

Darüber hinaus können die erfindungsgemäßen Zubereitungen die für kosmetische Sonnenschutzmittel üblichen Inhaltsstoffe in den üblichen Einsatzkonzentrationen enthalten.

**Vergleichsversuch**

Der erfindungsgemäße Effekt konnte mit dem folgenden Vergleichsversuch belegt werden. Es handelt sich dabei um identische Rezepturen, die sich allein im Gehalt an 2-Propylheptyloctanoat unterscheiden, dass gegen die entsprechende Menge (in Gewichts-%) Wasser ausgetauscht wurde.

Es wurden die folgenden Rezepturen hergestellt:

| IYFACE#68 | IYFACE#70 | INCI |
|---|---|---|
| 0,125 | 0,125 | Wirkstoffe (Glycyrrhetinic Acid, Glycyrrhiza Inflata Root Extract, Tocopheryl Acetate) |
| 3,46 | 3,44 | CI 77491, CI 77492, CI 77499, CI 77891 |
| 2,8 | 2,8 | Butylene Glycol Dicaprylate/Dicaprate + Dimethicone |
| | 1 | Propylheptyl Caprylate |
| 0,4 | 0,4 | Sodium Stearoyl Glutamate |
| 3 | 3 | Silica Dimethyl Silylate + Distarch Phosphate |
| 0,3 | 0,3 | Parfum |
| 3 | 3 | Glycerin + Aqua |
| 0,8 | 0,8 | Phenoxyethanol |
| 2,5 | 2,5 | Behenyl Alcohol + Cetearyl Alcohol |
| 0,55 | 0,55 | Carbomer, Acrylates/C10-30 Alkyl Acrylate Crosspolymer, Xanthan Gum |
| Ad. 100 | Ad. 100 | Aqua |
| 5 | 5 | Alcohol Denat. + Aqua |
| 1,7 | 1,7 | Aqua + Trisodium EDTA + Sodium Hydroxide |
| 9,5 | 9,5 | Homosalate, Ethylhexyl Salicylat |
| 3,5 | 3,5 | Bis-Ethylhexyloxyphenol Methoxyphenyl Triazine |
| 4 | 4 | Ethylhexyl Triazone + Phenylbenzimidazole Sulfonic Acid |

(fortgesetzt)

| IYFACE#68 | IYFACE#70 | INCI |
|---|---|---|
| 4,5 | 4,5 | Butyl Methoxydibenzoylmethane |

### *In-vitro* Sandanhaftung

50 mg der Test-Emulsion wurden auf PMMA Schönberg Platten (5,0 x 5,0 cm) aufgetragen und gleichmäßig mit einem Fingerling auf der Platte verteilt. Anschließend wird die aufgetragene Beispielrezeptur für 15 min bei Raumtemperatur getrocknet. Danach wurde das Gewicht der getrockneten Platten mit einer Analysenwaage ermittelt. Anschließend wurden die Platten mit feinem Seesand (1.07711.1000 Seesand reinst, der Firma Merck KGaA) im Überschuss übergossen. Durch einmaliges Rutschen der Platten auf einer dafür vorgesehenen Rutschvorrichtung (siehe unten) wurde lose anhaftender Sand mit reproduzierbarer, einheitlicher Kraft entfernt.

Der daraufhin auf der Platte zurückbleibende anhaftende Sand wurde durch Auswiegen ermittelt. Die Sandanhaftung kann mit folgender Gleichung ermittelt werden:

$$\Delta\left(Anhaftung\right)[mg] = m\left(Platte\ mit\ Sand\right)[mg] - m\left(eingecremte\ Platte\right)[mg]$$

Die Rutschvorrichtung ist eine in Form eines Dreiecks aufgebaute Konstruktion, bei der die Breite der Rutsche 5 cm beträgt. Die Rutschvorrichtung ist eine in Form eines rechtwinkligen Dreiecks aufgebaute Konstruktion, bei der die Breite der Rutsche 5 cm beträgt. Die Ankathete des rechtwinkeligen Dreiecks (= Standfläche der Rutsche) hat eine Länge von 13,5 cm, die Gegenkathete (= Fallhöhe) hat eine Länge von 49 cm. Die Hypotenuse, auf welcher der Rutschvorgang stattfindet, und die Standfläche schließen einen Winkel von 275 ° ein.

Die Versuche wurden je Rezeptur 10x wiederholt und der entsprechende Mittelwert gebildet.

| Ansatz | Platte ohne Sand | Platte mit Sand | Anhaftender Sand in g | Mittelwert | Standardabweichung |
|---|---|---|---|---|---|
| **IYFACE#68** | 7,233 | 7,606 | 0,373 | | |
| (ohne Propylheptyl Caprylate) | 7,180 | 7,607 | 0,427 | | |
| | 7,136 | 7,550 | 0,414 | | |
| | 7,227 | 7,629 | 0,402 | | |
| | 7,213 | 7,591 | 0,378 | | |
| | 7,155 | 7,487 | 0,332 | | |
| | 7,142 | 7,576 | 0,434 | | |
| | 7,156 | 7,611 | 0,455 | | |
| | 7,171 | 7,523 | 0,352 | | |
| | 7,134 | 7,463 | 0,329 | **0,390** | **0,044** |
| **IYFACE#70** | 7,167 | 7,473 | 0,306 | | |
| (mit 1% Propylheptyl Caprylate) | 7,137 | 7,497 | 0,360 | | |
| | 7,153 | 7,436 | 0,283 | | |
| | 7,149 | 7,445 | 0,296 | | |
| | 7,209 | 7,581 | 0,372 | | |
| | 7,230 | 7,541 | 0,311 | | |
| | 7,180 | 7,528 | 0,348 | | |
| | 7,220 | 7,533 | 0,313 | | |
| | 7,167 | 7,423 | 0,256 | | |
| | 7,127 | 7,472 | 0,345 | **0,319** | **0,037** |

(fortgesetzt)

| Ansatz | Platte ohne Sand | Platte mit Sand | Anhaftender Sand in g | Mittelwert | Standardabweichung |
|---|---|---|---|---|---|
| Ansatz | anhaftender Sand in mg/cm$^2$ | | | | |
| IYFACE #68 | 15,58 | | | | |
| IYFACE #70 | 12,76 | | | | |

**Beispiele**

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen. Diese Zubereitungen weisen nur eine geringe Sandanhaftung auf.

| 1 | 2 | 3 | 4 | 5 | 6 | INCI |
|---|---|---|---|---|---|---|
| 0,1 | 0,1 | 0,1 | 0,1 | 0,1 | 0,1 | Tocopheryl Acetate |
| 0,025 | 0,025 | 0,025 | 0,025 | 0,025 | 0,025 | Glycyrrhiza Inflata Root Extract |
| | | | 0,05 | | 0,05 | Magnolia Officinalis Bark Extract |
| | | | | 0,3 | | Ethylhexylglycerin |
| | | | | 0,03 | | Ubiquinone + Aqua |
| 0,27 | | 0,27 | 0,27 | | | CI 77492 + CI 77491 + CI 77499 |
| 0,08 | | 0,08 | 0,08 | | | CI 77491 + CI 77499 |
| 0,37 | | 0,37 | 0,37 | | | CI 77492 |
| 0,013 | | 0,013 | 0,013 | | | CI 77491 |
| 3,71 | | 3,71 | 3,71 | | | CI 77891 |
| 3 | 2 | 1,5 | 2 | 2 | 2 | Propylheptyl Caprylate |
| 0,8 | 0,8 | 0,8 | 0,8 | 0,8 | 0,8 | Dimethicone |
| | 1 | 1,5 | 1 | 3 | 1 | Butylene Glycol Dicaprylate/Dicaprate |
| | | 2 | 2 | | 2 | Dibutyl Adipate |
| | | 5 | 5 | 1 | 5 | C12-15 Alkyl Benzoate |
| 0,4 | 0,4 | 0,4 | 0,4 | 0,4 | 0,4 | Sodium Stearoyl Glutamate |
| | | | | 0,8 | | Glyceryl Stearate |
| 1 | 1 | 1 | 1 | | 1 | Silica Dimethyl Silylate |
| 3 | 4 | 2 | 3 | 2 | 3 | Distarch Phosphate |
| 0,3 | 0,3 | 0,3 | 0,3 | 0,3 | 0,3 | Parfum |
| 1 | 2 | 3 | 1 | 6 | 1 | Glycerin + Aqua |
| 0,7 | 0,7 | 0,65 | 0,65 | 0,92 | 0,65 | Aqua + Sodium Hydroxide |
| 0,8 | 0,8 | 0,8 | 0,9 | 0,7 | 0,9 | Phenoxyethanol |
| 1 | 1 | 1 | 1 | | 1 | Behenyl Alcohol |
| 1,5 | 1,5 | 1,5 | 1,5 | 2 | 1,5 | Cetearyl Alcohol |
| 0,2 | 0,2 | 0,2 | 0,2 | 0,1 | 0,2 | Carbomer |
| 0,15 | 0,15 | 0,15 | 0,15 | 0,15 | 0,15 | Acrylates/C10-30 Alkyl Acrylate Crosspolymer |
| 0,2 | 0,2 | 0,2 | 0,2 | 0,1 | 0,2 | Xanthan Gum |

(fortgesetzt)

| 1 | 2 | 3 | 4 | 5 | 6 | INCI |
|---|---|---|---|---|---|---|
| 53,882 | 57,325 | 54,932 | 54,782 | 49,275 | 59,225 | Aqua |
| 6 | 5 | 5 | 6 | 4 | 6 | Alcohol Denat. + Aqua |
| 1 | 1 | 1 | 1 | 1 | 1 | Aqua + Trisodium EDTA |
| 4 | 4 | | | 6 | | Homosalate |
| 4,5 | 4,5 | 4,5 | 4,5 | 4,75 | 4,5 | Ethylhexyl Salicylate |
| 3,5 | 3,5 | 2,5 | 2,5 | 3,5 | 2,5 | Bis-Ethylhexyloxyphenol Methoxyphenyl Triazine |
| 3 | 3 | 1,5 | 1,5 | 4 | 1,5 | Ethylhexyl Triazone |
| 4,5 | 4,5 | 3 | 3 | 4,75 | 3 | Butyl Methoxydibenzoylmethane |
| 1 | 1 | 1 | 1 | 2 | 1 | Phenylbenzimidazole Sulfonic Acid |

**Patentansprüche**

1. Verwendung von 2-Propylheptyloctanoat (INCI Propylheptyl Caprylate) in kosmetischen Zubereitungen zur Reduzierung der Sand anhaftenden Eigenschaften der Zubereitung.

2. Verwendung nach Anspruch 1, **dadurch gekennzeichnet, dass** die Zubereitung mindestens einen im UV-A Bereich absorbierenden organischen UV-Filter enthält.

3. Verwendung nach einem der vorhergehenden Ansprüche, **dadurch gekennzeichnet, dass** die Zubereitung Phenoxyethanol und/oder Ethanol enthält.

4. Kosmetische Zubereitung enthaltend

   a) 2-Propylheptyloctanoat (INCI Propylheptyl Caprylate) in einer Konzentration von 1 bis 5 Gewichts-%, bezogen auf das Gesamtgewicht der Zubereitung,
   b) Phenoxyethanol,
   c) Ethanol sowie
   d) mindestens einen im UV-A Bereich absorbierenden organischen UV-Filter, wobei die Zubereitung frei ist von 3-(4-Methylbenzyliden)-campher, 2-Hydroxy-4-methoxybenzophenon (INCI: Oxybenzon), Ethylhexyl-2-cyano-3,3-diphenylacrylat (INCI: Octocrylen), 4-Methoxyzimtsäure-2-ethylhexylester (INCI Octylmethoxycinnamate), Parabenen, Methylisothiazolinon, Chlormethylisothiazolinon und DMDM-Hydantoin, **dadurch gekennzeichnet, dass** die Zubereitung eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, β-Alanin, Panthenol, Magnolol, Honokiol, Tocopherylacetat, Dihydroxyaceton; 8-Hexadecen-1,16-dicarbonsäure, Glycerylglycose, (2-Hydroxyethyl)harnstoff, Vitamin E bzw. seine Derivate, Hyaluronsäure und/oder deren Salze und/oder Licochalcon A enthält.

5. Zubereitung nach Anspruch 4, **dadurch gekennzeichnet, dass** der oder die im UV-A Bereich absorbierenden organischen UV-Filter gewählt werden aus der Gruppe der Verbindungen 4-(tert.-Butyl)-4'-methoxydibenzoylmethan, Hexyl 2-[4-(diethylamino)-2-hydroxybenzoyl]benzoate (INCI: Diethylamino hydroxybenzoyl hexyl benzoate) und/oder 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine).

6. Zubereitung nach einem der vorhergehenden Ansprüche 4 oder 5, **dadurch gekennzeichnet, dass** die Zubereitung Phenoxyethanol in einer Konzentration von 0,2 bis 1,0 Gewichts-%, bezogen auf das Gesamtgewicht der Zubereitung, enthält.

7. Zubereitung nach einem der vorhergehenden Ansprüche 4 bis 6, **dadurch gekennzeichnet, dass** die Zubereitung Ethanol in einer Konzentration von 0,5 bis 20 Gewichts-%, bezogen auf das Gesamtgewicht der Zubereitung, enthält.

8. Zubereitung nach einem der vorhergehenden Ansprüche 4 bis 7, **dadurch gekennzeichnet, dass** die Zubereitung einen oder mehrere weitere UV-Filter gewählt aus der Gruppe der Verbindungen Salicylsäure-2-ethylhexylester (INCI Octyl Salicylate), 3,3,5-Trimethylcyclohexyl 2-hydroxybenzoat (INCI: Homosalate), 2,4,6-Tris-[anilino-(p-carbo-2'-ethyl-1'-hexyloxy)]-1,3,5-triazin (INCI: Ethylhexyl Triazone) und/oder 2-Phenylbenzimidazol-5-sulfonsäuresalze, enthält.

9. Zubereitung nach einem der vorhergehenden Ansprüche 4 bis 8, **dadurch gekennzeichnet, dass** die Zubereitung in Form einer O/W-Emulsion vorliegt.

10. Zubereitung nach einem der vorhergehenden Ansprüche 4 bis 9, **dadurch gekennzeichnet, dass** die Zubereitung Natriumstearylglutamat als Emulgator enthält.

11. Zubereitung nach einem der vorhergehenden Ansprüche 4 bis 10, **dadurch gekennzeichnet, dass** die Zubereitung ein oder mehrere Alkandiole aus der Gruppe der Verbindungen 1,2-Pentandiol, 1,2-Hexandiol, 1,2-Octandiol, 1,2-Decandiol, 2-Methyl-1,3-propandiol enthält.

12. Zubereitung nach einem der vorhergehenden Ansprüche 4 bis 11, **dadurch gekennzeichnet, dass** die Zubereitung eine oder mehrere Öle gewählt aus der Gruppe der Verbindungen Di-n-butyladipat, Dicaprylylcarbonat, C12-C15 Alkylbenzoat, Butylene Glycol und/oder Dicaprylate/Dicaprate, enthält.

13. Zubereitung nach einem der vorhergehenden Ansprüche 4 bis 12, **dadurch gekennzeichnet, dass** die Zubereitung keine Polyethylenglycolether oder Polyethylenglycolester enthält.

**Claims**

1. Use of 2-propylheptyl octanoate (INCI Propylheptyl Caprylate) in cosmetic preparations for reducing the sand-adhering properties of the preparation.

2. The use according to claim 1, wherein the preparation comprises at least one organic UV filter absorbing in the UVA range.

3. The use according to either of the preceding claims, wherein the preparation comprises phenoxyethanol and/or ethanol.

4. A cosmetic preparation comprising

    a) 2-propylheptyl octanoate (INCI Propylheptyl Caprylate) at a concentration of 1% to 5% by weight, based on the total weight of the preparation,
    b) phenoxyethanol,
    c) ethanol and
    d) at least one organic UV filter absorbing in the UVA range,

    the preparation being free of 3-(4-methylbenzylidene)camphor, 2-hydroxy-4-methoxybenzophenone (INCI: Oxybenzone), ethylhexyl 2-cyano-3,3-diphenylacrylate (INCI: Octocrylene), 2-ethylhexyl 4-methoxycinnamate (INCI Octylmethoxycinnamate), parabens, methylisothiazolinone, chloromethylisothiazolinone and DMDM hydantoin, wherein the preparation comprises one or more compounds selected from the group of compounds comprising alpha-lipoic acid, folic acid, phytoene, D-biotin, coenzyme Q10, alpha-glucosylrutin, carnitine, carnosine, natural and/or synthetic isoflavonoids, flavonoids, creatine, creatinine, taurine, β-alanine, panthenol, magnolol, honokiol, tocopheryl acetate, dihydroxyacetone; 8-hexadecene-1,16-dicarboxylic acid, glycerylglucose, (2-hydroxyethyl)urea, vitamin E and derivatives thereof, hyaluronic acid and/or salts thereof and/or licochalcone A.

5. The preparation according to claim 4, wherein the organic UV filter(s) absorbing in the UVA range are selected from the group of compounds comprising 4-(tert-butyl)-4'-methoxydibenzoylmethane, hexyl 2-[4-(diethylamino)-2-hydroxybenzoyl]benzoate (INCI: Diethylamino hydroxybenzoyl hexyl benzoate) and/or 2,4-bis-{[4-(2-ethylhexyloxy)-2-hydroxy]phenyl}-6-(4-methoxyphenyl)-1,3,5-triazine (INCI: Bis-ethylhexyloxyphenol methoxyphenyl triazine).

6. The preparation according to either of the preceding claims 4 and 5, wherein the preparation comprises phenoxyethanol at a concentration of 0.2% to 1.0% by weight, based on the total weight of the preparation.

7. The preparation according to any of the preceding claims 4 to 6, wherein the preparation comprises ethanol at a concentration of 0.5% to 20% by weight, based on the total weight of the preparation.

8. The preparation according to any of the preceding claims 4 to 7, wherein the preparation comprises one or more further UV filters selected from the group of compounds comprising 2-ethylhexyl salicylate (INCI Octyl Salicylate), 3,3,5-trimethylcyclohexyl 2-hydroxybenzoate (INCI: Homosalate), 2,4,6-tris[anilino-(p-carbo-2'-ethyl-1'-hexyloxy)]-1,3,5-triazine (INCI: Ethylhexyl Triazone) and/or 2-phenylbenzimidazole-5-sulfonic acid salts.

9. The preparation according to any of the preceding claims 4 to 8, wherein the preparation is in the form of an O/W emulsion.

10. The preparation according to any of the preceding claims 4 to 9, wherein the preparation comprises sodium stearoyl glutamate as emulsifier.

11. The preparation according to any of the preceding claims 4 to 10, wherein the preparation comprises one or more alkanediols from the group of compounds comprising 1,2-pentanediol, 1,2-hexanediol, 1,2-octanediol, 1,2-decanediol, 2-methyl-1,3-propanediol.

12. The preparation according to any of the preceding claims 4 to 11, wherein the preparation comprises one or more oils selected from the group of compounds comprising di-n-butyl adipate, dicaprylyl carbonate, C12-C15 alkyl benzoate, butylene glycol and/or dicaprylate/dicaprate.

13. The preparation according to any of the preceding claims 4 to 12, wherein the preparation does not comprise any polyethylene glycol ethers or polyethylene glycol esters.

**Revendications**

1. Utilisation d'octanoate de 2-propylheptyle (INCI : Propylheptyl Caprylate) dans des préparations cosmétiques pour la réduction des propriétés d'adhérence au sable de la préparation.

2. Utilisation selon la revendication 1, **caractérisée en ce que** la préparation contient au moins un filtre UV organique absorbant dans la plage UV-A.

3. Utilisation selon l'une quelconque des revendications précédentes, **caractérisée en ce que** la préparation contient du phénoxyéthanol et/ou de l'éthanol.

4. Préparation cosmétique contenant :

a) de l'octanoate de 2-propylheptyle (INCI : Propylheptyl Caprylate) en une concentration de 1 à 5 % en poids, par rapport au poids total de la préparation,
b) du phénoxyéthanol,
c) de l'éthanol, ainsi que
d) au moins un filtre UV organique absorbant dans la plage UV-A, la préparation étant exempte de 3-(4-méthylbenzylidène)-camphre, de 2-hydroxy-4-méthoxybenzophénone (INCI : Oxybenzon), d'acrylate d'éthylhexyl-2-cyano-3,3-diphényle (INCI : Octocrylen), d'ester 2-éthylhexylique de l'acide 4-méthoxycinnamique (INCI : Octylmethoxycinnamate), de parabènes, de méthylisothiazolinone, de chlorométhyl-isothiazolinone et de DMDM-hydantoïne, **caractérisée en ce que** la préparation contient un ou plusieurs composés choisis dans le groupe de composés composé par l'acide alpha-liponique, l'acide folique, le phytoène, la D-biotine, la coenzyme Q10, l'alpha-glucosylrutine, la carnitine, la carnosine, les isoflavonoïdes naturels et/ou synthétiques, les flavonoïdes, la créatine, la créatinine, la taurine, la β-alanine, le panthénol, le magnolol, l'honokiol, l'acétate de tocophéryle, la dihydroxyacétone ; l'acide 8-hexadécène-1,16-dicarboxylique, le glycérylglycose, la (2-hydroxyéthyl)urée, la vitamine E et ses dérivés, l'acide hyaluronique et/ou ses sels et/ou la licochalcone A.

5. Préparation selon la revendication 4, **caractérisée en ce que** le ou les filtres UV organiques absorbant dans la plage UV-A sont choisis dans le groupe de composés composé par le 4-(tert.-butyl)-4'-méthoxydibenzoylméthane, le 2-[4-(diéthylamino)-2-hydroxybenzoyl]benzoate d'hexyle (INCI : Diethylamino Hydroxybenzoyl Hexyl Benzoate) et/ou la 2,4-bis-{[4-(2-éthyl-hexyloxy)-2-hydroxy]-phényl}-6-(4-méthoxyphényl)-1,3,5-triazine (INCI: Bis-Ethylhexyloxyphenol Methoxyphenyl Triazine).

6. Préparation selon l'une quelconque des revendications 4 ou 5 précédentes, **caractérisée en ce que** la préparation contient du phénoxyéthanol en une concentration de 0,2 à 1,0 % en poids, par rapport au poids total de la préparation.

7. Préparation selon l'une quelconque des revendications 4 à 6 précédentes, **caractérisée en ce que** la préparation contient de l'éthanol en une concentration de 0,5 à 20 % en poids, par rapport au poids total de la préparation.

8. Préparation selon l'une quelconque des revendications 4 à 7 précédentes, **caractérisée en ce que** la préparation contient un ou plusieurs filtres UV supplémentaires, choisis dans le groupe de composés composé par l'ester 2-éthylhexylique de l'acide salicylique (INCI : Octyl Salicylate), le 2-hydroxybenzoate de 3,3,5-triméthylcyclohexyle (INCI : Homosalate), la 2,4,6-tris-[anilino-(p-carbo-2'-éthyl-1'-hexyloxy)]-1,3,5-triazine (INCI : Ethylhexyl Triazone) et/ou les sels de l'acide 2-phénylbenzimidazole-5-sulfonique.

9. Préparation selon l'une quelconque des revendications 4 à 8 précédentes, **caractérisée en ce que** la préparation se présente sous la forme d'une émulsion H/E.

10. Préparation selon l'une quelconque des revendications 4 à 9 précédentes, **caractérisée en ce que** la préparation contient du glutamate de stéaryle sodique en tant qu'émulsifiant.

11. Préparation selon l'une quelconque des revendications 4 à 10 précédentes, **caractérisée en ce que** la préparation contient un ou plusieurs alcane-diols du groupe de composés composé par le 1,2-pentanediol, le 1,2-hexane-diol, le 1,2-octane-diol, le 1,2-décane-diol, le 2-méthyl-1,3-propane-diol.

12. Préparation selon l'une quelconque des revendications 4 à 11 précédentes, **caractérisée en ce que** la préparation contient une ou plusieurs huiles choisies dans le groupe de composés composé par l'adipate de di-n-butyle, le carbonate de dicaprylyle, un benzoate d'alkyle en C12-C15, le butylène glycol et/ou des dicaprylates/dicaprates.

13. Préparation selon l'une quelconque des revendications 4 à 12 précédentes, **caractérisée en ce que** la préparation ne contient pas d'éther de polyéthylène glycol ou d'ester de polyéthylène glycol.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102007017436
- DE 102007017440
- EP 3093006 A1